# EUROPEAN PATENT SPECIFICATION

(11) **EP 3 434 269 B1**
(45) Date of publication and mention of the grant of the patent: **23.12.2020**
(21) Application number: 17770421.0
(22) Date of filing: 24.03.2017
(51) Int. Cl.: A61K 31/423, A61K 9/70, A61K 47/14, A61K 47/22, A61K 47/32, A61K 9/00

(54) **TRANSDERMAL ABSORPTION-TYPE PATCH PREPARATION COMPRISING ZONISAMIDE**
PFLASTERPRÄPARAT VOM TYP DER TRANSDERMALE ABSORPTION MIT ZONISAMID
PRÉPARATION PHARMACEUTIQUE SOUS FORME DE TIMBRE TRANSDERMIQUE À ABSORPTION PERCUTANÉE COMPRENANT UN ZONISAMIDE

(30) Priority: 25.03.2016 JP 2016062531
(43) Date of publication of application: 30.01.2019
(73) Proprietor: Teikoku Seiyaku Co., Ltd., Higashikagawa-shi, Kagawa 769-2695 (JP); Sumitomo Dainippon Pharma Co., Ltd., Osaka-shi Osaka 541-8524 (JP)
(72) Inventor: INOO, Katsuyuki, Higashikagawa-shi Kagawa 769-2695 (JP); TAKANO, Daiki, Higashikagawa-shi Kagawa 769-2695 (JP)
(74) Representative: Müller-Boré & Partner Patentanwälte PartG mbB
(86) International application number: PCT/JP2017/012055
(87) International publication number: WO 2017/164381

(56) References cited:
- WO-A1-2012/105625
- WO-A1-2013/128562
- WO-A1-2014/021393
- WO-A1-2014/021393
- JP-A- 2005 529 150
- KARANDE P ET AL: "Enhancement of transdermal drug delivery via synergistic action of chemicals", BIOCHIMICA ET BIOPHYSICA ACTA (BBA) - BIOMEMBRANES, ELSEVIER, AMSTERDAM, NL, vol. 1788, no. 11, 1 November 2009 (2009-11-01), pages 2362-2373, XP026708909, ISSN: 0005-2736, DOI: 10.1016/J.BBAMEM.2009.08.015 [retrieved on 2009-09-03]

## Description

The present invention relates to a transdermal absorption-type patch preparation comprising an adhesive layer including zonisamide or an alkali metal salt thereof, an adhesive agent, and a transdermal absorption promoter.

Zonisamide (chemical name: 3-sulfamoylmethyl-1,2-benzisoxazole or 1,2-benzisoxazole-3-methanesulfonamide) is currently widely used as a therapeutic agent for child or adult epileptic seizure (partial seizure, generalized seizure, mixed seizure) or as a therapeutic agent for Parkinson's disease in various countries around the world. In Japan, it is used as a prophylactic drug for epileptic seizure under the trade name of EXCEGRAN (registered trademark), and an orally administered drug is provided as a therapeutic agent for Parkinson's disease under the trade name of TRERIEF (registered trademark).

In recent years, a transdermal absorption-type patch preparation comprising zonisamide has been studied. Patent Document 1 discloses a transdermal absorption-type preparation comprising zonisamide containing lactic acid and an absorption promoter such as α-monoisostearyl glyceryl ether and lauromacrogol. In addition, Patent Document 2 discloses a transdermal absorption-type preparation comprising zonisamide containing a specific ether type additive.

### PATENT DOCUMENTS

Patent Document 1: WO-A-2012/105625
Patent Document 2: WO-A-2014/021393

In general, a transdermal absorption-type patch preparation is superior to an oral dosage formulation in that it can sustainably maintain blood drug concentration and can be administered to patients who have difficulty in swallowing, and the like. On the other hand, it is difficult to send an effective amount of a transdermal absorption-type patch preparation into the body depending on the drug type, since the drug needs to be administered while avoiding the barrier function of the skin. Also, in order to impart high transdermal absorbability, it is necessary to contain many drugs in a dissolved state in the preparation. However, depending on the base components such as an absorption promoter and an adhesive agent, crystals of the drug precipitate in the adhesive agent during preservation of the preparation, or the like, so it is difficult to maintain the stability. On the other hand, when an absorption promoter is added to the preparation in expectation of the effect of weakening the barrier function of the skin, side effects such as skin irritation often occur.

While the transdermal absorption-type patch preparations described in Patent Document 1 and Patent Document 2 have certain transdermal absorbability, a transdermal absorption-type patch preparation further improved in safety and transdermal absorbability is desired.

An object of the present invention is to provide a transdermal absorption-type patch preparation comprising zonisamide, that is a transdermal absorption-type patch preparation comprising zonisamide or an alkali metal salt thereof, being capable of maintaining a high concentration of a drug in a dissolved state in the preparation and exhibiting sufficient and sustained drug efficacy. Furthermore, an object of the present invention is to provide a transdermal absorption-type patch preparation comprising zonisamide having low skin irritation and high safety even for long-term administration.

As a result of intensive studies, the present inventors have found that, by a combined use of a (meth)acrylic copolymer having a pyrrolidone group as an adhesive agent (hereinafter sometimes referred to as a pyrrolidone group-containing (meth)acrylic copolymer) with a specific N-alkylpyrrolidone as a transdermal absorption promoter, it is possible to maintain a high concentration of zonisamide in a dissolved state for a long time and realize a sufficient and sustained transdermal absorbability, thereby completing the present invention.

Furthermore, by containing a (meth)acrylic copolymer having a carboxyl group (hereinafter sometimes referred to as a carboxyl group-containing (meth)acrylic copolymer) in the adhesive agent, skin irritation was suppressed and high safety was realized over a long time, without reducing the permeability of the drug.

That is, the present invention is as follows.
[Item 1] A transdermal absorption-type patch preparation comprising an adhesive layer, the adhesive layer comprising
   zonisamide or an alkali metal salt thereof,
   an adhesive agent comprising a (meth)acrylic copolymer having a pyrrolidone group, and
   a transdermal absorption promoter comprising N-alkylpyrrolidone,
   wherein the N-alkylpyrrolidone is N-laurylpyrrolidone, N-octylpyrrolidone, N-heptylpyrrolidone, N-hexylpyrrolidone, N-nonylpyrrolidone, N-decylpyrrolidone, N-undecylpyrrolidone, N-tridecylpyrrolidone, N-tetradecylpyrrolidone, N-pentadecylpyrrolidone, N-hexadecylpyrrolidone, N-heptadecylpyrrolidone, or N-octadecylpyrrolidone.
[Item 2] The transdermal absorption-type patch preparation according to item 1, wherein the N-alkylpyrrolidone is N-laurylpyrrolidone or N-octylpyrrolidone.
[Item 3] The transdermal absorption-type patch preparation according to item 2, wherein the N-alkylpyrrolidone is N-laurylpyrrolidone.
[Item 4] The transdermal absorption-type patch preparation according to any one of items 1 to 3, wherein the adhesive agent comprises a (meth)acrylic copolymer having a pyrrolidone group and a (meth)acrylic copolymer having a carboxyl group.
[Item 5] The transdermal absorption-type patch preparation according to any one of items 1 to 4, wherein the transdermal absorption promoter comprises N-laurylpyrrolidone, and
   at least one member selected from the group consisting of polyethylene glycol monolaurate, lauromacrogol, isopropyl myristate, isopropyl palmitate, oleyl oleate, and hexyl laurate.
[Item 6] The transdermal absorption-type patch preparation according to item 5, wherein the transdermal absorption promoter comprises N-laurylpyrrolidone, and
   at least one member selected from the group consisting of polyethylene glycol monolaurate, and isopropyl myristate.
[Item 7] The transdermal absorption-type patch preparation according to item 6, wherein the transdermal absorption promoter comprises N-laurylpyrrolidone and isopropyl myristate.
[Item 8] The transdermal absorption-type patch preparation according to any one of items 1 to 7, wherein the (meth)acrylic copolymer having a pyrrolidone group is a 2-ethylhexyl acrylate-vinylpyrrolidone copolymer, a hydroxyethyl acrylate-vinylpyrrolidone copolymer, a butyl acrylate-vinylpyrrolidone copolymer, a cyclohexyl acrylate-vinylpyrrolidone copolymer, a 2-ethylhexyl methacrylate-vinylpyrrolidone copolymer, a cyclohexyl methacrylate-vinylpyrrolidone copolymer, a 2-ethylhexyl acrylate-methylvinylpyrrolidone copolymer, a 2-ethylhexyl acrylate-hydroxyethyl acrylate-vinylpyrrolidone copolymer, a 2-ethylhexyl acrylate-acrylic acid-vinylpyrrolidone copolymer, a butyl acrylate-acrylic acid-vinylpyrrolidone copolymer or a 2-ethylhexyl acrylate-acrylic acid-methylvinylpyrrolidone copolymer.
[Item 9] The transdermal absorption-type patch preparation according to item 8, wherein the (meth)acrylic copolymer having a pyrrolidone group is a 2-ethylhexyl acrylate-vinylpyrrolidone copolymer, a hydroxyethyl acrylate-vinylpyrrolidone copolymer, a butyl acrylate-vinylpyrrolidone copolymer, a 2-ethylhexyl methacrylate-vinylpyrrolidone copolymer, a 2-ethylhexyl acrylate-methylvinylpyrrolidone copolymer, a 2-ethylhexyl acrylate-hydroxyethyl acrylate-vinylpyrrolidone copolymer, or a 2-ethylhexyl acrylate-acrylic acid-vinylpyrrolidone copolymer.
[Item 10] The transdermal absorption-type patch preparation according to item 9, wherein the (meth)acrylic copolymer having a pyrrolidone group is a 2-ethylhexyl acrylate-vinylpyrrolidone copolymer.
[Item 11] The transdermal absorption-type patch preparation according to any one of items 4 to 10, wherein the (meth)acrylic copolymer having a carboxyl group is an acrylic acid-octyl acrylate copolymer, a 2-ethylhexyl acrylate-vinyl acetate-acrylic acid copolymer, a 2-ethylhexyl acrylate-methyl acrylate-acrylic acid-glycidyl methacrylate copolymer, a 2-ethylhexyl acrylate-acrylic acid-vinylpyrrolidone copolymer, a 2-ethylhexyl acrylate-acrylic acid-methylvinylpyrrolidone copolymer, a butyl acrylate-acrylic acid-vinylpyrrolidone copolymer, an acrylic acid-butyl acrylate copolymer, an acrylic acid-hydroxyethyl acrylate copolymer, or a 2-ethylhexyl acrylate-vinyl acetate-butyl acrylate-acrylic acid copolymer.
[Item 12] The transdermal absorption-type patch preparation according to any one of items 4 to 10, wherein the (meth)acrylic copolymer having a carboxyl group is an acrylic acid-octyl acrylate copolymer, a 2-ethylhexyl acrylate-vinyl acetate-acrylic acid copolymer, a 2-ethylhexyl acrylate-methyl acrylate-acrylic acid-glycidyl methacrylate copolymer, an acrylic acid-butyl acrylate copolymer, an acrylic acid-hydroxyethyl acrylate copolymer, or a 2-ethylhexyl acrylate-vinyl acetate-butyl acrylate-acrylic acid copolymer.
[Item 13] The transdermal absorption-type patch preparation according to item 11, wherein the (meth)acrylic copolymer having a carboxyl group is an acrylic acid-octyl acrylate copolymer, or a 2-ethylhexyl acrylate-vinyl acetate-acrylic acid copolymer.
[Item 14] The transdermal absorption-type patch preparation according to item 13, wherein the (meth)acrylic copolymer having a carboxyl group is an acrylic acid-octyl acrylate copolymer.
[Item 15] The transdermal absorption-type patch preparation according to any one of items 1 to 14, wherein a content of the zonisamide or the alkali metal salt thereof in the transdermal absorption-type patch preparation is 1% by mass to 20% by mass, based on a total amount of the adhesive layer in terms of zonisamide.
[Item 16] The transdermal absorption-type patch preparation according to item 15, wherein a content of the zonisamide or the alkali metal salt thereof in the transdermal absorption-type patch preparation is 3% by mass to 10% by mass, based on a total amount of the adhesive layer in terms of zonisamide.
[Item 17] The transdermal absorption-type patch preparation according to any one of items 1 to 14, wherein a content of the adhesive agent in the transdermal absorption-type patch preparation is 30% by mass to 98% by mass, based on the total amount of the adhesive layer.
[Item 18] The transdermal absorption-type patch preparation according to any one of items 1 to 14, 16, and 17, wherein a content of the adhesive agent in the transdermal absorption-type patch preparation is 60% by mass to 85% by mass, based on the total amount of the adhesive layer.
[Item 19] The transdermal absorption-type patch preparation according to any one of items 1 to 14 and 16 to 18, wherein the content of the (meth)acrylic copolymer having a pyrrolidone group in the transdermal absorption-type patch preparation is 50% by mass to 85% by mass, based on the total amount of the adhesive layer.
[Item 20] The transdermal absorption-type patch preparation according to any one of items 1 to 18, wherein the content of the (meth)acrylic copolymer having a pyrrolidone group in the transdermal absorption-type patch preparation is 50% by mass to 70% by mass, based on the total amount of the adhesive layer.
[Item 21] The transdermal absorption-type patch preparation according to any one of items 1 to 16 and 20, wherein the content of the carboxyl group-containing (meth)acrylic copolymer in the transdermal absorption-type patch preparation is 1% by mass to 20% by mass, based on the total amount of the adhesive layer.
[Item 22] The transdermal absorption-type patch preparation according to any one of items 1 to 16 and 18 to 21, wherein the content of the carboxyl group-containing (meth)acrylic copolymer in the transdermal absorption-type patch preparation is 5% by mass to 15% by mass, based on the total amount of the adhesive layer.
[Item 23] The transdermal absorption-type patch preparation according to any one of items 1 to 16, 21 and 22, wherein the content of the transdermal absorption promoter in the transdermal absorption-type patch preparation is 1% by mass to 40% by mass, based on the total amount of the adhesive layer.
[Item 24] The transdermal absorption-type patch preparation according to any one of items 1 to 16 and 21 to 23, wherein the content of the transdermal absorption promoter in the transdermal absorption-type patch preparation is 25% by mass to 35% by mass, based on the total amount of the adhesive layer.
[Item 25] The transdermal absorption-type patch preparation according to any one of items 1 to 16 and 20 to 24, wherein the content of the N-alkylpyrrolidone in the transdermal absorption-type patch preparation is 1% by mass to 30% by mass, based on the total amount of the adhesive layer.
[Item 26] The transdermal absorption-type patch preparation according to any one of items 1 to 16 and 20 to 25, wherein the content of the N-alkylpyrrolidone in the transdermal absorption-type patch preparation is 13% by mass to 20% by mass, based on the total amount of the adhesive layer.
[Item 27] The transdermal absorption-type patch preparation according to any one of items 1 to 26, wherein the compounding amount of the adhesive agent in the transdermal absorption-type patch preparation is 1.5 parts by mass to 40 parts by mass, based on 1 part by mass of the zonisamide or the alkali metal salt thereof.
[Item 28] The transdermal absorption-type patch preparation according to item 27, wherein the compounding amount of the adhesive agent in the transdermal absorption-type patch preparation is 10 parts by mass to 25 parts by mass, based on 1 part by mass of the zonisamide or the alkali metal salt thereof.
[Item 29] The transdermal absorption-type patch preparation according to any one of items 1 to 28, wherein the compounding amount of the transdermal absorption promoter in the transdermal absorption-type patch preparation is 0.05 parts by mass to 40 parts by mass, based on 1 part by mass of the zonisamide or the alkali metal salt thereof.
[Item 30] The transdermal absorption-type patch preparation according to any one of items 1 to 29, wherein the compounding amount of the transdermal absorption promoter in the transdermal absorption-type patch preparation is 0.1 parts by mass to 30 parts by mass, based on 1 part by mass of the zonisamide or the alkali metal salt thereof.
[Item 31] The transdermal absorption-type patch preparation according to item 30, wherein the compounding amount of the transdermal absorption promoter in the transdermal absorption-type patch preparation is 1 part by mass to 10 parts by mass, based on 1 part by mass of the zonisamide or the alkali metal salt thereof.
[Item 32] The transdermal absorption-type patch preparation according to item 1, wherein the adhesive agent comprises a (meth)acrylic copolymer having a pyrrolidone group,
   the transdermal absorption promoter comprises N-laurylpyrrolidone, and
   the compounding amount of the adhesive agent is 1.5 parts by mass to 40 parts by mass, and the compounding amount of the transdermal absorption promoter is 1 part by mass to 30 parts by mass, based on 1 part by mass of the zonisamide or the alkali metal salt thereof.
[Item 33] The transdermal absorption-type patch preparation according to item 1, wherein the adhesive agent comprises a 2-ethylhexyl acrylate-vinylpyrrolidone copolymer,
   the transdermal absorption promoter comprises N-laurylpyrrolidone, and
   the compounding amount of the adhesive agent is 5 parts by mass to 30 parts by mass, and the compounding amount of the transdermal absorption promoter is 1 part by mass to 20 parts by mass, based on 1 part by mass of the zonisamide or the alkali metal salt thereof.
[Item 34] The transdermal absorption-type patch preparation according to item 1, wherein the adhesive agent comprises a 2-ethylhexyl acrylate-vinylpyrrolidone copolymer,
   the transdermal absorption promoter comprises N-laurylpyrrolidone and isopropyl myristate, and the compounding amount of the adhesive agent is 10 parts by mass to 30 parts by mass, and the compounding amount of the transdermal absorption promoter is 1 part by mass to 15 parts by mass, based on 1 part by mass of the zonisamide or the alkali metal salt thereof.
[Item 35] The transdermal absorption-type patch preparation according to item 1, wherein the adhesive agent comprises a 2-ethylhexyl acrylate-vinylpyrrolidone copolymer and an acrylic acid-octyl acrylate copolymer, and
   the transdermal absorption promoter comprises N-laurylpyrrolidone and isopropyl myristate, and the compounding amount of the adhesive agent is 10 parts by mass to 25 parts by mass, and the compounding amount of the transdermal absorption promoter is 1 part by mass to 10 parts by mass, based on 1 part by mass of the zonisamide or the alkali metal salt thereof.
[Item 36] The transdermal absorption-type patch preparation according to item 1, wherein the adhesive agent comprises a 2-ethylhexyl acrylate-vinylpyrrolidone copolymer and an acrylic acid-octyl acrylate copolymer,
   the transdermal absorption promoter comprises N-laurylpyrrolidone and isopropyl myristate, and the compounding amount of the 2-ethylhexyl acrylate-vinylpyrrolidone copolymer is 10 parts by mass to 20 parts by mass, the compounding amount of the acrylic acid-octyl acrylate copolymer is 1 part by mass to 3 parts by mass, the compounding amount of the N-laurylpyrrolidone is 1 part by mass to 5 parts by mass, and the compounding amount of the isopropyl myristate is 1 part by mass to 5 parts by mass, based on 1 part by mass of the zonisamide or the alkali metal salt thereof.
[Item 37] The transdermal absorption-type patch preparation according to any one of items 1 to 36, wherein the thickness of the adhesive layer (layer containing zonisamide or an alkali metal salt thereof, a transdermal absorption promoter, an adhesive agent, and other additives as necessary) is 30 µm to 200 µm.

Since the transdermal absorption-type patch preparation comprising zonisamide of the present invention is composed of a combination of a specific adhesive agent and a specific transdermal absorption promoter, it is safe and can exhibit excellent transdermal absorbability for a long time.

Hereinafter, preferred embodiments of the present invention will be described in detail.

### 1. Zonisamide or alkali metal salt thereof

In the present invention, zonisamide (chemical name: 3-sulfamoylmethyl-1,2-benzisoxazole or 1,2-benzisoxazole-3-methanesulfonamide) or an alkali metal salt thereof is used. Example preferably includes zonisamide.

Examples of the alkali metal salt of zonisamide include sodium salts, potassium salts, and lithium salts, preferably include sodium salts and potassium salts, and more preferably include sodium salts. Zonisamide can be produced, for example, according to the methods described in JP-B-60-33114, JP-B-61-59288, and US 4,172,896.

Crystal polymorphism may exist in zonisamide or an alkali metal salt thereof, and zonisamide in the present invention includes those in any crystal form.

Since the zonisamide of the present invention may exist in one or both forms of a hydrate and a solvate, one or both of a hydrate and a solvate of zonisamide or an alkali metal salt thereof are also included in the compound of the present invention.

Dissolution of a drug in the present invention means a state in which the drug is all dissolved in the preparation, and also a state in which a part of the drug is dissolved.

The content of zonisamide or an alkali metal salt thereof (the value in terms of zonisamide in the case of zonisamide alkali metal salt) in the transdermal absorption-type patch preparation of the present invention is grasped as % by mass when the total amount of the adhesive layer is 100% by mass, and it is not particularly limited as far as formulation is possible. The content of zonisamide or an alkali metal salt thereof in the transdermal absorption-type patch preparation is preferably 1% by mass to 20% by mass, more preferably 3% by mass to 20% by mass, further preferably 3% by mass to 15% by mass, and most preferably 3% by mass to 10% by mass.

The lower limit of the content of zonisamide or an alkali metal salt thereof in the transdermal absorption-type patch preparation is preferably 1% by mass or more, and more preferably 3% by mass or more.

The upper limit of the content of zonisamide or an alkali metal salt thereof in the transdermal absorption-type patch preparation is preferably 20% by mass or less, more preferably 15% by mass or less, further preferably 10% by mass or less, and further more preferably 6% by mass or less.

When the content of zonisamide or an alkali metal salt thereof is less than 1% by mass, sustained transdermal absorption effect cannot be obtained. On the other hand, when the content of zonisamide or an alkali metal salt thereof exceeds 20% by mass, the solubility of the drug may be insufficient.

### 2. Adhesive agent

The adhesive agent that is a base component of the transdermal absorption-type patch preparation of the present invention contains a (meth)acrylic copolymer having a pyrrolidone group. Zonisamide is a poorly soluble drug, and when the adhesive agent does not have a pyrrolidone group, sufficient solubility of the drug cannot be obtained. By using a pyrrolidone group-containing (meth)acrylic copolymer, the solubility of zonisamide in the adhesive agent can be improved.

The content of the adhesive agent in the transdermal absorption-type patch preparation of the present invention is preferably 30% by mass to 98% by mass, more preferably 40% by mass to 95% by mass, further preferably 50% by mass to 90% by mass, and most preferably 60% by mass to 85% by mass, when the total amount of the adhesive layer is 100% by mass.

The compounding amount of the adhesive agent in the transdermal absorption-type patch preparation of the present invention is preferably a ratio of 1.5 parts by mass to 40 parts by mass, more preferably a ratio of 5 parts by mass to 30 parts by mass, further preferably a ratio of 10 parts by mass to 30 parts by mass, and most preferably a ratio of 10 parts by mass to 25 parts by mass, based on 1 part by mass of zonisamide or an alkali metal salt thereof.

The pyrrolidone group-containing (meth)acrylic copolymer of the present invention is not particularly limited as long as it has a pyrrolidone group. The (meth)acrylic copolymer having a pyrrolidone group is a copolymer of at least one (meth)acrylate ester monomer and at least one monomer having a pyrrolidone group (hereinafter sometimes referred to as pyrrolidone group-containing monomer).

The pyrrolidone group-containing (meth)acrylic copolymer may have a carboxyl group.

Examples of the (meth)acrylate ester monomer include acrylic acids, methacrylic acids, or derivatives thereof. These can be used singly or in combination of two or more kinds. Examples of the derivatives include acrylate esters or methacrylate esters.

Specific examples of the acrylate esters are preferably n-butyl acrylate, n-hexyl acrylate, n-octyl acrylate, 2-ethylhexyl acrylate, isooctyl acrylate, isononyl acrylate, n-decyl acrylate, isodecyl acrylate, hydroxyethyl acrylate, and cyclohexyl acrylate.

Specific examples of the methacrylate esters are preferably n-decyl methacrylate, dodecyl methacrylate, 2-ethylhexyl methacrylate, isodecyl methacrylate, lauryl methacrylate, cyclohexyl methacrylate, and the like.

Incidentally, each of the (meth)acrylate esters can be used singly or in combination of two or more kinds.

A specific example of the pyrrolidone group-containing monomer is preferably vinylpyrrolidone or methylvinyl pyrrolidone, and more preferably N-vinyl-2 -pyrrolidone.

Incidentally, each of the pyrrolidone group-containing monomers can be used singly or in combination of two or more kinds.

A specific example of the (meth)acrylic copolymer having a pyrrolidone group is preferably a 2-ethylhexyl acrylate-vinylpyrrolidone copolymer, a hydroxyethyl acrylate-vinylpyrrolidone copolymer, a butyl acrylate-vinylpyrrolidone copolymer, a cyclohexyl acrylate-vinylpyrrolidone copolymer, a 2-ethylhexyl methacrylate-vinylpyrrolidone copolymer, a cyclohexyl methacrylate-vinylpyrrolidone copolymer, a 2-ethylhexyl acrylate-methylvinylpyrrolidone copolymer, a 2-ethylhexyl acrylate-hydroxyethyl acrylate-vinylpyrrolidone copolymer, a 2-ethylhexyl acrylate-acrylic acid-vinylpyrrolidone copolymer, a butyl acrylate-acrylic acid-vinylpyrrolidone copolymer, or an 2-ethylhexyl acrylate-acrylic acid-methylvinylpyrrolidone copolymer.

A specific example of the (meth)acrylic copolymer having a pyrrolidone group is more preferably a 2-ethylhexyl acrylate-vinylpyrrolidone copolymer, a hydroxyethyl acrylate-vinylpyrrolidone copolymer, a butyl acrylate-vinylpyrrolidone copolymer, a 2-ethylhexyl methacrylate-vinylpyrrolidone copolymer, a 2-ethylhexyl acrylate-methylvinylpyrrolidone copolymer, a 2-ethylhexyl acrylate-hydroxyethyl acrylate-vinylpyrrolidone copolymer, or a 2-ethylhexyl acrylate-acrylic acid-vinyl pyrrolidone copolymer.

A specific example of the (meth)acrylic copolymer having a pyrrolidone group is further preferably a 2-ethylhexyl acrylate-vinylpyrrolidone copolymer.

As the adhesive agent of the pyrrolidone group-containing (meth)acrylic copolymer used in the present invention, a commercially available product can be used. Specific examples include acrylic adhesive agents such as MAS683 manufactured by CosMED Pharmaceutical Co. Ltd.

The content (the sole content when contained singly, and the total amount when contained in combination of two or more kinds of components) of the pyrrolidone group-containing (meth)acrylic copolymer in the transdermal absorption-type patch preparation of the present invention is preferably 30% by mass to 98% by mass, more preferably 40% by mass to 95% by mass, further preferably 50% by mass to 90% by mass, further more preferably 50% by mass to 85% by mass, and most preferably 50% by mass to 70% by mass, when the total amount of the adhesive layer is 100% by mass.

The lower limit of the content of the pyrrolidone group-containing (meth)acrylic copolymer in the transdermal absorption-type patch preparation is preferably 30% by mass or more, more preferably 40% by mass or more, and further preferably 50% by mass or more.

The upper limit of the content of the pyrrolidone group-containing (meth)acrylic copolymer in the transdermal absorption-type patch preparation is preferably 98% by mass or less, more preferably 95% by mass or less, further preferably 90% by mass or less, further more preferably 85% by mass or less, and most preferably 70% by mass or less.

When the content of the pyrrolidone group-containing (meth)acrylic copolymer is less than 30% by mass, a problem that the cohesive force of the adhesive layer is not sufficient so that the adhesive agent remains on the skin, or a problem such as precipitation of crystals due to decrease in the solubility of the drug is caused. On the other hand, when the content of the pyrrolidone group-containing (meth)acrylic copolymer exceeds 98% by mass, the drug concentration decreases and the drug releasing property from the preparation decreases, so that sufficient transdermal absorbability of the drug cannot be obtained.

The compounding amount (the sole content when contained singly, and the total amount when contained in combination of two or more kinds) of the pyrrolidone group-containing (meth)acrylic copolymer as an adhesive agent in the transdermal absorption-type patch preparation of the present invention is preferably a ratio of 1.5 parts by mass to 40 parts by mass, more preferably a ratio of 5 parts by mass to 30 parts by mass, further preferably a ratio of 10 parts by mass to 30 parts by mass, and most preferably a ratio of 10 parts by mass to 20 parts by mass, based on 1 part by mass of zonisamide or an alkali metal salt thereof.

The adhesive agent used in the present invention preferably contains a carboxyl group-containing (meth)acrylic copolymer, in addition to the (meth)acrylic copolymer having a pyrrolidone group. Depending on the type or addition amount of the transdermal absorption promoter, skin irritation is a concern, but by allowing an appropriate amount of the carboxyl group-containing (meth)acrylic copolymer to coexist, it is possible to improve the transdermal absorbability of the drug, and also suppress the skin irritation due to the absorption promoter. The carboxyl group-containing (meth)acrylic copolymer is not particularly limited as long as it has a carboxyl group, and it may have a carboxyl group in the copolymerization counterpart component of the acrylic acid. Example of the carboxyl group-containing (meth)acrylic copolymer includes a copolymer of at least two or more (meth)acrylic acid monomers, or a copolymer of at least one (meth)acrylic acid monomer and vinyl acetate monomer.

Examples of the (meth)acrylic acid monomer include acrylic acids, methacrylic acids, or derivatives thereof. Specific examples of the derivatives include (meth)acrylate ester monomers.

Specific examples of the acrylate esters are preferably n-butyl acrylate, n-hexyl acrylate, n-octyl acrylate, 2-ethylhexyl acrylate, isooctyl acrylate, isononyl acrylate, n-decyl acrylate, isodecyl acrylate, and hydroxyethyl acrylate.

Specific examples of the methacrylate esters include n-decyl methacrylate, dodecyl methacrylate, 2-ethylhexyl methacrylate, isodecyl methacrylate, lauryl methacrylate, glycidyl methacrylate, and the like.

A specific example of the (meth)acrylate ester monomer is preferably 2-ethylhexyl acrylate, octyl acrylate, butyl acrylate, cyclohexyl acrylate, octyl methacrylate, 2-ethylhexyl methacrylate, butyl methacrylate, or cyclohexyl methacrylate.

Incidentally, each of the (meth)acrylate esters can be used singly or in combination of two or more kinds.

A specific example of the carboxyl group-containing (meth)acrylic copolymer is preferably an acrylic acid-octyl acrylate copolymer, a 2-ethylhexyl acrylate-vinyl acetate-acrylic acid copolymer, a 2-ethylhexyl acrylate-methyl acrylate-acrylic acid-glycidyl methacrylate copolymer, a 2-ethylhexyl acrylate-acrylic acid-vinylpyrrolidone copolymer, a 2-ethylhexyl acrylate-acrylic acid-methylvinylpyrrolidone copolymer, a butyl acrylate-acrylic acid-vinylpyrrolidone copolymer, an acrylic acid-butyl acrylate copolymer, an acrylic acid-hydroxyethyl acrylate copolymer, or a 2-ethylhexyl acrylate-vinyl acetate-butyl acrylate-acrylic acid copolymer.

A specific example of the carboxyl group-containing (meth)acrylic copolymer is more preferably an acrylic acid-octyl acrylate copolymer, a 2-ethylhexyl acrylate-vinyl acetate-acrylic acid copolymer, a 2-ethylhexyl acrylate-methyl acrylate-acrylic acid-glycidyl methacrylate copolymer, an acrylic acid-butyl acrylate copolymer, an acrylic acid-hydroxyethyl acrylate copolymer, or a 2-ethylhexyl acrylate-vinyl acetate-butyl acrylate-acrylic acid copolymer.

A more specific example of the carboxyl group-containing (meth)acrylic copolymer is further preferably an acrylic acid-octyl acrylate copolymer, or a 2-ethylhexyl acrylate-vinyl acetate-acrylic acid copolymer.

A specific example of the carboxyl group-containing (meth)acrylic copolymer is further more preferably an acrylic acid-octyl acrylate copolymer.

Incidentally, those classified into both a pyrrolidone group-containing (meth)acrylic copolymer and a carboxyl group-containing (meth)acrylic copolymer may be used as a pyrrolidone group-containing (meth)acrylic copolymer or may be used as a carboxyl group-containing (meth)acrylic copolymer.

As the adhesive agent of the carboxyl group-containing (meth)acrylic copolymer used in the present invention, a commercially available product can be used. A specific example of the carboxyl group-containing (meth)acrylic copolymer includes Duro-Tak 87-200A, Duro-Tak 87-2194, Duro-Tak 87-2353, Duro-Tak 87-2051, Duro-Tak 87-235A, or the like produced by Henkel.

The content (the sole content when contained singly, and the total amount when contained in combination of two or more kinds) of the carboxyl group-containing (meth)acrylic copolymer in the transdermal absorption-type patch preparation of the present invention is preferably 20% by mass or less, more preferably 1% by mass to 20% by mass, further preferably 5% by mass to 20% by mass, and further more preferably 5% by mass to 15% by mass, when the total amount of the adhesive layer is 100% by mass.

The lower limit of the content of the carboxyl group-containing (meth)acrylic copolymer in the transdermal absorption-type patch preparation is preferably 1% by mass or more, and more preferably 5% by mass or more.

The upper limit of the content of the carboxyl group-containing (meth)acrylic copolymer in the transdermal absorption-type patch preparation is preferably 20% by mass or less, and more preferably 15% by mass or less.

When the content of the carboxyl group-containing (meth)acrylic copolymer is less than 1% by mass, the suppression effect on the skin irritation of the absorption promoter is lowered. On the other hand, when the content of the carboxyl group-containing (meth)acrylic copolymer exceeds 20% by mass, undesirable effects such as relative decrease in the content of the pyrrolidone group-containing (meth)acrylic copolymer, decrease in the solubility of the drug, and precipitation of crystals occur.

The compounding amount (the sole content when contained singly, and the total amount when contained in combination of two or more kinds) of the (meth)acrylic copolymer having a carboxyl group as an adhesive agent in the transdermal absorption-type patch preparation of the present invention is preferably a ratio of 20 parts by mass or less, more preferably a ratio of 10 parts by mass or less, further preferably a ratio of 0.05 part by mass to 5 parts by mass, and most preferably a ratio of 1 part by mass to 3 parts by mass, based on 1 part by mass of zonisamide or an alkali metal salt thereof.

### 3. Transdermal absorption promoter

The transdermal absorption-type patch preparation of the present invention contains a specific N-alkylpyrrolidone as a transdermal absorption promoter. By using such N-alkylpyrrolidone, sufficient and sustained drug transdermal absorption can be realized.

According to the present invention, the N-alkylpyrrolidone is N-laurylpyrrolidone, N-octylpyrrolidone, N-heptylpyrrolidone, N-hexylpyrrolidone, N-nonylpyrrolidone, N-decylpyrrolidone, N-undecylpyrrolidone, N-tridecylpyrrolidone, N-tetradecylpyrrolidone, N-pentadecylpyrrolidone, N-hexadecylpyrrolidone, N-heptadecylpyrrolidone, or N-octadecylpyrrolidone, more preferably N-laurylpyrrolidone or N-octylpyrrolidone, and further preferably N-laurylpyrrolidone.

The transdermal absorption promoter in the transdermal absorption-type patch preparation of the present invention is preferably 1% by mass to 40% by mass, more preferably 5% by mass to 40% by mass, further preferably 15% by mass to 35% by mass, and most preferably 25% by mass to 35% by mass, when the total amount of the adhesive layer is 100% by mass.

The compounding amount of the transdermal absorption promoter in the transdermal absorption-type patch preparation of the present invention is preferably a ratio of 0.05 parts by mass to 40 parts by mass, more preferably a ratio of 0.1 parts by mass to 30 parts by mass, further preferably a ratio of 0.5 parts by mass to 30 parts by mass, further more preferably a ratio of 1 part by mass to 30 parts by mass, still more preferably a ratio of 1 part by mass to 20 parts by mass, still further preferably, a ratio of 1 part by mass to 15 parts by mass, and most preferably a ratio of 1 part by mass to 10 parts by mass, based on 1 part by mass of zonisamide or an alkali metal salt thereof.

The content (the sole content when contained singly, and the total amount when contained in combination of two or more kinds) of N-alkylpyrrolidone in the transdermal absorption-type patch preparation of the present invention is preferably 1% by mass to 30% by mass, more preferably 5% by mass to 30% by mass, further preferably 13% by mass to 25% by mass, and most preferably 13% by mass to 20% by mass, when the total amount of the adhesive layer is 100% by mass.

The lower limit of the content of N-alkylpyrrolidone in the transdermal absorption-type patch preparation is preferably 1% by mass or more, more preferably 5% by mass or more, and further preferably 13% by mass or more.

The upper limit of the content of N-alkylpyrrolidone in the transdermal absorption-type patch preparation is preferably 30% by mass or less, more preferably 25% by mass or less, and further preferably 20% by mass or less.

When the content of N-alkylalkyl pyrrolidone is less than 1% by mass, sufficient transdermal absorbability of the drug cannot be obtained. On the other hand, when the content of N-alkylpyrrolidone exceeds 30% by mass, problems of skin irritation such as redness and edema may occur.

The compounding amount (the sole content when contained singly, and the total amount when contained in combination of two or more kinds) of N-alkylpyrrolidone as a transdermal absorption promoter in the transdermal absorption-type patch preparation of the present invention is preferably a ratio of 1 part by mass to 30 parts by mass, more preferably a ratio of 1 part by mass to 20 parts by mass, further preferably a ratio of 1 part by mass to 10 parts by mass, and most preferably a ratio of 1 part by mass to 5 parts by mass, based on 1 part by mass of zonisamide or an alkali metal salt thereof.

The transdermal absorption promoter used in the present invention may further contain an absorption promoter other than N-alkylpyrrolidone (hereinafter sometimes referred to as other absorption promoter). Specific examples of other absorption promoter are preferably at least one member selected from the group consisting of polyethylene glycol monolaurate, lauromacrogol, isopropyl myristate, isopropyl palmitate, oleyl oleate, hexyl laurate, diethyl sebacate, diisopropyl adipate, propylene glycol, dipropylene glycol, POE hydrogenated castor oil, sorbitan monooleate, sorbitan monolaurate, POE stearyl ether, and PEG monostearate, more preferably at least one member selected from the group consisting of polyethylene glycol monolaurate, lauromacrogol, isopropyl myristate, isopropyl palmitate, oleyl oleate, and hexyl laurate, further preferably at least one member selected from the group consisting of polyethylene glycol monolaurate and isopropyl myristate, and most preferably, isopropyl myristate is contained.

The content (the sole content when contained singly, and the total amount when contained in combination of two or more kinds) of the other absorption promoter in the transdermal absorption-type patch preparation of the present invention is preferably 1% by mass to 30% by mass, more preferably 5% by mass to 30% by mass, further preferably 10% by mass to 30% by mass, and most preferably 10% by mass to 20% by mass, when the total amount of the adhesive layer is 100% by mass.

The lower limit of the content of the other absorption promoter in the transdermal absorption-type patch preparation is preferably 1% by mass or more, more preferably 5% by mass or more, and further preferably 10% by mass or more.

The upper limit of the content of the other absorption promoter in the transdermal absorption-type patch preparation is preferably 30% by mass or less, and more preferably 20% by mass or less.

When the content of the other absorption promoter is less than 1% by mass, sufficient transdermal absorbability of the drug is not obtained in some cases. On the other hand, when the content of the other absorption promoter exceeds 30% by mass, problems of skin irritation such as redness and edema may occur, and due to deterioration of the physical properties of the preparation, adhesive residue or the like may occur after application of the preparation.

The compounding amount (the sole content when contained singly, and the total amount when contained in combination of two or more kinds) of the other absorption promoter in the transdermal absorption-type patch preparation of the present invention is preferably a ratio of 0.1 parts by mass to 20 parts by mass, further preferably a ratio of 1 part by mass to 10 parts by mass, and most preferably a ratio of 1 part by mass to 5 parts by mass, based on 1 part by mass of zonisamide or an alkali metal salt thereof.

When an additive described below is added, the content of zonisamide or an alkali metal salt thereof, a transdermal absorption promoter, an adhesive agent, and the like shall be construed as % by mass based on the entire adhesive layer to which the additive is added (not including a support and a release liner).

### 4. Other

The preparation of the present invention includes an adhesive layer containing the zonisamide or the alkali metal salt thereof, the transdermal absorption promoter, and the adhesive agent. In the preparation of the present invention, in addition to the above, an additive usually used in transdermal absorption-type patch preparations can be included as necessary within the range not impairing the effect of the present invention.

For example, in order to adjust adhesiveness and stability of an adhesive base using the acrylic adhesive agent as the adhesive base, the preparation of the present invention can contain a softening agent such as polyisobutylene, polybutene or liquid paraffin, a water soluble polymer such as polyvinyl pyrrolidone or polyvinyl alcohol, a cellulose derivative such as ethylcellulose, hydroxypropylcellulose or hydroxypropylmethylcellulose, a silicon compound such as silicic acid anhydride or light anhydrous silicic acid, an inorganic filler such as silicas, and an antioxidant such as dibutylhydroxytoluene in appropriate amounts. Further, as necessary, appropriate amounts of a preservative, a cooling agent, a fungicide, a flavoring agent, and a coloring agent may be contained.

The components constituting the preparation of the present invention have been described above.

The transdermal absorption-type patch preparation of the present invention can be used as a laminated structure prepared by laminating an adhesive composition containing the zonisamide or the alkali metal salt thereof, the transdermal absorption promoter, and the adhesive agent described above as an adhesive layer on a support, and coating a release liner on the adhesive layer. Upon use, the release liner is peeled off, and the surface of the adhesive layer may be attached to a desired skin.

The support used in the present invention is not particularly limited, and a stretchable or non-stretchable one usually used for a patch is used. A specific example of the support is preferably a film or sheet formed by a synthetic resin such as polyethylene terephthalate (hereinafter sometimes referred to as PET), polyethylene, polypropylene, polybutadiene, ethylene vinyl acetate copolymer, polyvinyl chloride, polyester, nylon or polyurethane, a laminated body of these, a porous membrane, a foam, a woven fabric, a nonwoven fabric, or a paper material.

The release liner used in the transdermal absorption-type patch preparation of the present invention is not particularly limited as long as it is usually used for a patch. Specific examples of the release liner are preferably polyethylene terephthalate, polypropylene, paper, and the like, and more preferably polyethylene terephthalate. In order to optimize the peeling force of the release liner, silicon treatment may be carried out as necessary.

The transdermal absorption-type patch preparation of the present invention can be produced, for example, by the following method.

Zonisamide or an alkali metal salt thereof, a transdermal absorption promoter, and an adhesive agent (other additives component as necessary) are dissolved in an appropriate solvent to obtain an adhesive solution. As the solvent, suitable one may be appropriately selected according to the type of constituent components, and for example, toluene, ethyl acetate, N-methyl-2-pyrrolidone, ethanol, methanol or the like can be used singly or in a mixture of two or more kinds. Next, the adhesive solution thus obtained is spread on a release liner or a support to dry and remove the solvent, and then attached to a support or a release liner, whereby the transdermal absorption-type patch preparation of the present invention can be obtained.

The thickness of the adhesive layer (the layer containing the zonisamide or the alkali metal salt thereof, the transdermal absorption promoter, the adhesive agent, and other additives as necessary) is preferably 30 µm to 200 µm, more preferably 50 µm to 200 µm, and further preferably, 50 µm to 150 µm.

When the thickness of the adhesive layer is less than 30 µm, sustainability of drug release decreases. The thickness of the adhesive layer is preferably 30 µm or more, and more preferably 50 µm or more. On the other hand, when the thickness of the adhesive layer exceeds 200 µm, the drug content in the adhesive layer increases, the amount of remaining drug increases, and the production cost increases. The thickness of the adhesive layer is preferably 200 µm or less, and more preferably 150 µm or less.

### EXAMPLES

Hereinbelow, the present invention will be more specifically described with reference to examples, but the present invention is not limited to the following examples. In the following description, "%" means "% by mass" when the total amount of the adhesive layer is 100% by mass, unless otherwise specified.

### Study 1-1: Study on Various Absorption Promoters

The following experiments were carried out using zonisamide as zonisamide or an alkali metal salt thereof.

### Example 1

4 g of zonisamide and 7.5 g of N-laurylpyrrolidone (N-lauryl-2-pyrrolidone) were dissolved in 20 mL of N-methyl-2-pyrrolidone to prepare a main drug solution. The main drug solution and 295 g of an ethyl acetate solution in which 88.5 g of a pyrrolidone group-containing (meth)acrylic copolymer (2-ethylhexyl acrylate-vinylpyrrolidone copolymer) was dissolved were mixed, then ethyl acetate was added to a total amount of 400 g, and the mixture was stirred until homogeneous to obtain an adhesive solution. Next, an appropriate amount of this adhesive solution was spread on a release liner (PET film), and then N-methyl-2-pyrrolidone and ethyl acetate were removed by drying to form an adhesive layer with a thickness of 50 µm. Subsequently, a support (PET film) was laminated to obtain Example 1. Table 1 shows the content of each component. In Table 1, blanks indicate not added.

### Comparative Example 1

Comparative Example 1 was obtained by the same production method as in Example 1, except that N-laurylpyrrolidone was not used and the content of the pyrrolidone group-containing (meth)acrylic copolymer was changed to the content shown in Table 1. Table 1 shows the content of each component.

### Comparative Examples 2 to 4

Comparative Examples 2 to 4 were obtained by the same production method as in Example 1, except that the transdermal absorption promoter shown in Table 1 was used in place of N-laurylpyrrolidone. Table 1 shows the content of each component.

### Comparative Examples 5 to 13

Comparative Examples 5 to 13 were obtained by the same production method as in Example 1, except that the transdermal absorption promoter shown in Table 1 was used in place of N-laurylpyrrolidone and the content of the pyrrolidone group-containing (meth)acrylic copolymer and the transdermal absorption promoter was changed to the content shown in Table 1. Table 1 shows the content of each component.

### Example 2

Example 2 was obtained by the same production method as in Example 1, except that the content of the pyrrolidone group-containing (meth)acrylic copolymer and the N-laurylpyrrolidone was changed to the content shown in Table 2. Table 2 shows the content of each component. In Table 2, blanks indicate not added.

### Comparative Examples 14 to 19

Comparative Examples 14 to 19 were obtained by the same production method as in Example 1, except that the transdermal absorption promoter shown in Table 2 was used in place of the N-laurylpyrrolidone and the content of the pyrrolidone group-containing (meth)acrylic copolymer and the transdermal absorption promoter was changed to the content shown in Table 2. Table 2 shows the content of each component.

### In Vitro Hairless Rat Skin Permeability Test of Zonisamide

In order to study the transdermal absorbability of zonisamide in the transdermal absorption-type patch preparation of the present invention, in vitro skin permeability test in a hairless rat was performed on the preparations of Examples 1 and 2 and Comparative Examples 1 to 19. Excised abdominal skin of male hairless rat (HWY series, 7 weeks old) was put in a Franz diffusion cell, and each test preparation cut into a round shape (ϕ 14 mm) was applied. The receptor side was filled with phosphate buffered saline and warm water at 37°C was circulated in a water jacket. A receptor solution was sampled with time, the content of zonisamide permeated the skin was measured by liquid chromatography, and the cumulative drug permeation amount 24 hours after the start of the test was calculated. The conditions of the liquid chromatography method are as follows.

### [HPLC Measurement Conditions]

Column: ACQUITY BEH C18 column (particle diameter 1.7 µm, inner diameter × length; 2.1 × 100 mm)
Flow rate: 0.4 mL/min
Column temperature: 30°C
Wavelength: 285 nm
Mobile phase: Methanol/water/acetic acid = 18:82:1

The results are shown in Tables 1 and 2.

**[Table 1]**

| | | Example **1** | Comparative Example 1 | Comparative Example 2 | Comparative Example 3 | Comparative Example 4 | Comparative Example 5 | Comparative Example 6 | Comparative Example 7 | Comparative Example 8 | Comparative Example 9 | Comparative Example 10 | Comparative Example 11 | Comparative Example 12 | Comparative Example 13 |
|---|---|---|---|---|---|---|---|---|---|---|---|---|---|---|---|
| Drug (% by mass) | Zonisamide | 4 | 4 | 4 | 4 | 4 | 4 | 4 | 4 | 4 | 4 | 4 | 4 | 4 | 4 |
| Adhesive agent (% by mass) | 2-ethylhexyl acrylate-vinylpyrrolidone copolymer | 88.5 | 96.0 | 88.5 | 88.5 | 88.5 | 86.0 | 86.0 | 86.0 | 86.0 | 86.0 | 86.0 | 86.0 | 86.0 | 86.0 |
| Transdermal absorption promoter (% by mass) | N-lauryl-2-pyrrolidone | 7.5 | | | | | | | | | | | | | |
| | Polyethylene glycol monolaurate | | | | | | 10 | | | | | | | | |
| | Isopropyl myristate | | | | | | | 10 | | | | | | | |
| | Oleyl oleate | | | 7.5 | | | | | | | | | | | |
| | Diethyl sebacate | | | | 7.5 | | | | | | | | | | |
| | Diisopropyl adipate | | | | | 7.5 | | | | | | | | | |
| | Propylene glycol | | | | | | | | 10 | | | | | | |
| | Dipropylene glycol | | | | | | | | | 10 | | | | | |
| | POE hydrogenated castor oil | | | | | | | | | | 10 | | | | |
| | Sorbitan monooleate | | | | | | | | | | | 10 | | | |
| | Sorbitan monolaurate | | | | | | | | | | | | 10 | | |
| | POE stearyl ether | | | | | | | | | | | | | 10 | |
| | PEG monostearate | | | | | | | | | | | | | | 10 |
| Cumulative drug permeation amount 24 hours after start of test (*µ* g/cm²) | | 50.0 | 10.9 | 38.0 | 20.5 | 15.5 | 40.5 | 31.3 | 12.7 | 14.8 | 18.1 | 21.2 | 20.0 | 22.1 | 16.3 |

**[Table 2]**

| | | Example 2 | Comparative Example 14 | Comparative Example 15 | Comparative Example 16 | Comparative Example 17 | Comparative Example 18 | Comparative Example 19 |
|---|---|---|---|---|---|---|---|---|
| Drug (% by mass) | Zonisamide | 4 | 4 | 4 | 4 | 4 | 4 | 4 |
| Adhesive agent (% by mass) | 2-ethylhexyl acrylate-vinylpyrrolidone copolymer | 81 | 81 | 81 | 81 | 81 | 81 | 81 |
| Transdermal absorption promoter (% by mass) | N-lauryl-2-pyrrolidone | 15 | | | | | | |
| | Lauromacrogol (BL-9EX) | | 15 | | | | | |
| | Isopropyl myristate | | | 15 | | | | |
| | Isopropyl palmitate | | | | 15 | | | |
| | Oleyl oleate | | | | | 15 | | |
| | Diethyl sebacate | | | | | | 15 | |
| | Diisopropyl adipate | | | | | | | 15 |
| Cumulative drug permeation amount 24 hours after start of test (*µ*g/cm²) | | 125.3 | 72.0 | 45.0 | 56.0 | 45.4 | 27.2 | 26.2 |

Based on Tables 1 and 2, it can be considered as follows.

Example 1 in Table 1 is an example satisfying a constitutional requirement of the present invention, and Comparative Examples 1 to 13 are examples in which a transdermal absorption promoter (N-alkylpyrrolidone) specified in the present invention is not used, or examples in which other transdermal absorption promoter is used. In Example 1, the cumulative drug permeation amount greatly increased more than in Comparative Examples 1 to 13, and excellent transdermal absorbability was exhibited.

Among them, Comparative Examples 5 to 13 are examples in which the content of the transdermal absorption promoter is 10% by mass and the content of the transdermal absorption promoter is higher than that of Example 1 (7.5% by mass). Example 1 exhibited transdermal absorbability of about 1.2 to 4 times that of Comparative Examples 5 to 13, although the content of the transdermal absorption promoter was smaller than that of Comparative Examples 5 to 13.

Example 2 in Table 2 is an example satisfying the constitutional requirement of the present invention, and Comparative Examples 14 to 19 are examples in which other transdermal absorption promoter is used without using the transdermal absorption promoter (N-alkylpyrrolidone) specified in the present invention. Example 2 exhibited transdermal absorbability of about 1.7 to 4.8 times that of Comparative Examples 14 to 19 using other transdermal absorption promoter.

Based on the above, in the present invention, it is insufficient to only use a pyrrolidone group-containing (meth)acrylic copolymer as an adhesive agent, and it is important to use N-laurylpyrrolidone (N-alkylpyrrolidone) as a transdermal absorption promoter for improving transdermal absorbability.

Study 1-2: Study of Combination of N-laurylpyrrolidone and Other Absorption Promoter, and Study of Combination of Pyrrolidone Group-Containing (Meth)acrylic Copolymer and Carboxyl Group-Containing (Meth)acrylic Copolymer

### Examples 3 to 7

Examples 3 to 7 were obtained by the same production method as in Example 1, except that the transdermal absorption promoter shown in Table 3 was used and the content of the pyrrolidone group-containing (meth)acrylic copolymer and the transdermal absorption promoter was changed to the content shown in Table 3. Table 3 shows the content of each component. In Table 3, blanks indicate not added.

### Comparative Example 20

Comparative Example 20 was obtained by the same production method as in Example 1, except that the transdermal absorption promoter shown in Table 3 was used in place of N-laurylpyrrolidone and the content of the pyrrolidone group-containing (meth)acrylic copolymer and the transdermal absorption promoter was changed to the content shown in Table 3. Table 3 shows the content of each component.

### Examples 8 to 11

Examples 8 to 11 were obtained by the same production method as in Example 1, except that the transdermal absorption promoter shown in Table 4 was used and the content of zonisamide, the pyrrolidone group-containing (meth)acrylic copolymer, and the transdermal absorption promoter was changed to the content shown in Table 4. Table 4 shows the content of each component. In Table 4, blanks indicate not added.

### Example 12

Example 12 was obtained by the same production method as in Example 1, except that a carboxyl group-containing (meth)acrylic copolymer (an acrylic acid-octyl acrylate copolymer) was used and the content of zonisamide, the acrylic copolymer, and the transdermal absorption promoter was changed to the content shown in Table 4. Table 4 shows the content of each component.

### Examples 13 to 21

Examples 13 to 21 were obtained by the same production method as in Example 12, except that the transdermal absorption promoter shown in Table 4 was used and the content of the acrylic copolymer and the transdermal absorption promoter was changed to the content shown in Table 4. Table 4 shows the content of each component.

### Comparative Example 21

Comparative Example 21 was obtained by the same production method as in Example 1, except that the transdermal absorption promoter shown in Table 4 was used in place of N-laurylpyrrolidone and the content of zonisamide, the pyrrolidone group-containing (meth)acrylic copolymer, and the transdermal absorption promoter was changed to the content shown in Table 4. Table 4 shows the content of each component.

### In Vitro Hairless Rat Skin Permeability Test of Zonisamide

In vitro skin permeability test in hairless rats was performed on the preparations of Examples 3 to 21 and Comparative Examples 20 and 21, in the same manner as the above in vitro hairless rat skin permeability test. The results are shown in Tables 3 and 4.

**[Table 3]**

| | | Example 3 | Example 4 | Example 5 | Example 6 | Example 7 | Comparative Example 20 |
|---|---|---|---|---|---|---|---|
| Drug (% by mass) | Zonisamide | 4 | 4 | 4 | 4 | 4 | 4 |
| Adhesive agent (% by mass) | 2-ethylhexyl acrylate-vinylpyrrolidone copolymer | 66 | 66 | 66 | 66 | 66 | 66 |
| Transdermal absorption promoter (% by mass) | N-lauryl-2-pyrrolidone | 15 | 15 | 15 | 15 | 15 | |
| | Polyethylene glycol monolaurate | 15 | | | | | 15 |
| | Lauromacrogol (BL-9EX) | | | | | | 15 |
| | Isopropyl myristate | | 15 | | | | |
| | Isopropyl palmitate | | | 15 | | | |
| | Oleyl oleate | | | | 15 | | |
| | Hexyl laurate | | | | | 15 | |
| | Amount of transdermal absorption promoter (% by mass) | 30 | 30 | 30 | 30 | 30 | 30 |
| Cumulative drug permeation amount 24 hours after start of test (*µ*g/cm²) | | 254.4 | 268.1 | 261.2 | 227.0 | 248.9 | 164.2 |

**[Table 4]**

| | | | Example 8 | Example 9 | Example 10 | Example 11 | Example 12 | Example 13 | Example 14 | Example 15 | Example 16 | Example 17 | Example 18 | Example 19 | Example 20 | Example 21 | Comparativ e Example 21 |
|---|---|---|---|---|---|---|---|---|---|---|---|---|---|---|---|---|---|
| Drug (% by mass) | | Zonisamide | 5 | 5 | 5 | 5 | 5 | 5 | 5 | 5 | 5 | 5 | 5 | 5 | 5 | 5 | 5 |
| Adhesive agent (% by mass) | Pyrrolidone group-containing | 2-ethylhexyl acrylate-vinylpyrrolidone copolymer | 65 | 65 | 65 | 65 | 65 | 65 | 60 | 60 | 55 | 55 | 54 | 53 | 51 | 49 | 65 |
| | Carboxyl group-containing | Acrylic acid-octyl acrylate copolymer | | | | | 10 | 10 | 10 | 10 | 10 | 10 | 10 | 10 | 10 | 10 | |
| Transdermal absorption promoter (% by mass) | | N-lauryl-2-pyrrolidone | 15.0 | 15.0 | 15.0 | 15.0 | 20.0 | 15.0 | 15.0 | 20.0 | 20.0 | 15.0 | 15.0 | 15.0 | 15.0 | 15.0 | |
| | | Polyethylene glycol monolaurate | 4 | 8 | 12 | | | | | | | | 1 | 2 | 4 | 6 | 15 |
| | | Isopropyl myristate | 11.0 | 7.0 | 3.0 | 15.0 | | 5.0 | 10.0 | 5.0 | 10.0 | 15.0 | 15.0 | 15.0 | 15.0 | 15.0 | |
| | | Lauromacrogol (BL-9EX) | | | | | | | | | | | | | | | 15 |
| | | Amount of transdermal absorption promoter (% by mass) | 30 | 30 | 30 | 30 | 20 | 20 | 25 | 25 | 30 | 30 | 31 | 32 | 34 | 36 | 30 |
| Cumulative drug permeation amount 24 hours after start of test (*µ*g/cm²) | | | 306.8 | 328.3 | 316.3 | 283.1 | 288.5 | 229.8 | 268.9 | 309.3 | 321.8 | 308.6 | 325.8 | 351.5 | 382.5 | 397.5 | 167.8 |

Based on Tables 3 and 4, it can be considered as follows.

Examples 3 to 7 in Table 3 are examples satisfying the constitutional requirement of the present invention in which the transdermal absorption promoter (N-alkylpyrrolidone) specified in the present invention and other transdermal absorption promoter are used. Also, Comparative Example 20 in Table 3 is an example in which two types of other transdermal absorption promoters were used without using the transdermal absorption promoter specified in the present invention. In Examples 3 to 7, the cumulative drug permeation amount increased more than in Comparative Example 20, and excellent transdermal absorbability was exhibited. Among them, in Example 4 in which isopropyl myristate was used as other transdermal absorption promoter, the cumulative drug permeation amount was the highest.

Based on the above, it became clear that it is also effective to use N-laurylpyrrolidone and other transdermal absorption promoter in the present invention. In particular, it became clear that the combination of N-laurylpyrrolidone and isopropyl myristate is effective for improving transdermal absorbability.

Examples 8 to 11 in Table 4 are examples satisfying the constitutional requirement of the present invention in which the transdermal absorption promoter (N-alkylpyrrolidone) specified in the present invention and one or two other transdermal absorption promoters are used. Also, Comparative Example 21 in Table 4 is an example in which two types of other transdermal absorption promoters were used without using the transdermal absorption promoter specified in the present invention. In Examples 8 to 11, the cumulative drug permeation amount increased more than in Comparative Example 21, and excellent transdermal absorbability was exhibited.

Example 12 in Table 4 is an example satisfying the constitutional requirement of the present invention in which a pyrrolidone group-containing (meth)acrylic copolymer and a carboxyl group-containing (meth)acrylic copolymer are used as adhesive agents and the transdermal absorption promoter (N-alkylpyrrolidone) specified in the present invention is further used. In Example 12, the cumulative drug permeation amount increased more than in Comparative Example 21, and excellent transdermal absorbability was exhibited.

Example 13 to 21 in Table 4 are examples satisfying the constitutional requirement of the present invention in which a pyrrolidone group-containing (meth)acrylic copolymer and a carboxyl group-containing (meth)acrylic copolymer are used as adhesive agents and the transdermal absorption promoter (N-alkylpyrrolidone) specified in the present invention and one or two other transdermal absorption promoters are further used. In Examples 13 to 21, the cumulative drug permeation amount increased more than in Comparative Example 21, and excellent transdermal absorbability was exhibited.

When comparing Example 11 and Example 17 in Table 4, in Example 17 containing a carboxyl group-containing (meth)acrylic copolymer, the cumulative drug permeation amount increased more than in Example 11, and excellent transdermal absorbability was exhibited.

Based on the above, it became clear that it is also effective to use a carboxyl group-containing (meth)acrylic copolymer in addition to the pyrrolidone group-containing (meth)acrylic copolymer as adhesive agents, for improving transdermal absorbability in the present invention.

### Study 2: Study on Skin Irritation

### Comparative Example 22

Comparative Example 22 was obtained by the same production method as in Example 1, except that the transdermal absorption promoter shown in Table 5 was used in place of N-laurylpyrrolidone and the content of the pyrrolidone group-containing (meth)acrylic copolymer and the transdermal absorption promoter was changed to the content shown in Table 5. Table 5 shows the content of each component. In Table 5, blanks indicate not added.

### Rabbit Skin Primary Irritation Test

Rabbit skin primary irritation test was performed on each of the preparations of Examples 2, 11, and 17, and Comparative Examples 1 and 22. Each patch was applied to the depilated back of a rabbit for 24 hours, and primary irritation index (P.I.I.) was obtained from an average of the sum of erythema scores and edema scores at 1 hour, 24 hours, and 48 hours after peeling, according to the following Draize method. The P.I.I. values are shown in Table 5. Also, safety evaluation criteria are shown in Table 6. The P.I.I. value of Example 11 was taken as a reference value since adhesive residues after peeling occurred.

### (Evaluation criteria and scores)

### Formation of erythema and scab

No erythema: 0
Very mild erythema (barely discernible): 1
Definite erythema: 2
Moderate to severe erythema: 3
Severe erythema to mild scab formation (deep damage): 4 Formation of edema
No edema: 0
Very mild edema (barely discernible): 1
Mild edema (defined edge due to definite bulge is discernible): 2
Moderate edema (bulge of about 1 mm): 3
Severe edema (bulge of 1 mm or more and spread beyond exposure range): 4

**[Table 5]**

| | | | Example2 | Example 1 | Example1 7 | Comparative Example 1 | Comparative Example 22 |
|---|---|---|---|---|---|---|---|
| Drug (% by mass) | | Zonisamide | 4 | 5 | 5 | 4 | 4 |
| Adhesive agent (% by mass) | Pyrrolidone group-containing | 2-ethylhexyl acrylate-vinylpyrrolidone copolymer | 81 | 65 | 55 | 96 | 81 |
| | Carboxyl group-containing | Acrylic acid-octyl acrylate copolymer | | | 10 | | |
| Transdermal absorption promoter (% by mass) | | N-lauryl-2-pyrrolidone | 15 | 15 | 15 | | |
| | | Isopropyl myristate | | 15 | 15 | | |
| | | Lauromacrogol (BL-4. 2) | | | | | 15 |
| Rabbit Skin Primary Irritation Test Adhesive residues at peeling | | | Not occurred | Occurred | Not occurred | Not occurred | Not occurred |
| Rabbit Skin Primary Irritation [P. I.I] | | | 2.1 | *1.9 | 0.9 | 1.5 | 5.8 |

| | | | | | | | |
|---|---|---|---|---|---|---|---|
| *The value was taken as a reference value since adhesive residues occurred | | | | | | | |

**[Table 6]**

| (Evaluation criteria) | |
|---|---|
| P.I.I | Safety classification |
| P.I.I=0 | Non irritation |
| O<P.I.I<2 | Low irritation |
| 2≦P.I.I<5 | Medium irritation |
| 5≦P.I.I | Strong irritation |

Examples 2, 11, and 17 in Table 5 are examples satisfying the constitutional requirement of the present invention. Comparative Example 22 in Table 5 is an example in which lauromacrogol (BL-4.2) was used in place of the transdermal absorption promoter (the N-laurylpyrrolidone) specified in the present invention. It became clear that, while Comparative Example 22 containing lauromacrogol (BL-4.2) had an irritation index of 5.8, Examples 2 and 11 of the present invention had irritation indices of around 2.0, respectively, which are high in safety, though the content of the transdermal absorption promoter was the same as or more than that in Comparative Example 22.

Furthermore, it became clear that Example 17 containing the carboxyl group-containing acrylic adhesive agent had an irritation index of 0.9 which is extremely high in safety. As shown in Table 4, Example 17 has better transdermal absorbability than Example 11, and by containing a carboxyl group-containing acrylic adhesive agent, transdermal absorbability was improved and skin irritation could be reduced.

While Comparative Example 1 that does not contain a transdermal absorption promoter had an irritation index of 1.5, Example 17 had an irritation index of 0.9, and increased in safety more than the preparation that does not contain a transdermal absorption promoter, by containing a carboxyl group-containing acrylic adhesive agent.

## Claims

1. A transdermal absorption-type patch preparation comprising an adhesive layer, the adhesive layer comprising
zonisamide or an alkali metal salt thereof,
an adhesive agent comprising a (meth)acrylic copolymer having a pyrrolidone group, and
a transdermal absorption promoter comprising N-alkylpyrrolidone,
wherein the N-alkylpyrrolidone is N-laurylpyrrolidone, N-octylpyrrolidone, N-heptylpyrrolidone, N-hexylpyrrolidone, N-nonylpyrrolidone, N-decylpyrrolidone, N-undecylpyrrolidone, N-tridecylpyrrolidone, N-tetradecylpyrrolidone, N-pentadecylpyrrolidone, N-hexadecylpyrrolidone, N-heptadecylpyrrolidone, or N-octadecylpyrrolidone.

2. The transdermal absorption-type patch preparation according to claim 1, wherein the N-alkylpyrrolidone is N-laurylpyrrolidone or N-octylpyrrolidone.

3. The transdermal absorption-type patch preparation according to claim 2, wherein the N-alkylpyrrolidone is N-laurylpyrrolidone.

4. The transdermal absorption-type patch preparation according to any one of claims 1 to 3, wherein the adhesive agent comprises a (meth)acrylic copolymer having a pyrrolidone group and a (meth)acrylic copolymer having a carboxyl group.

5. The transdermal absorption-type patch preparation according to any one of claims 1 to 4, wherein the transdermal absorption promoter comprises N-laurylpyrrolidone, and
at least one member selected from the group consisting of polyethylene glycol monolaurate, lauromacrogol, isopropyl myristate, isopropyl palmitate, oleyl oleate, and hexyl laurate.

6. The transdermal absorption-type patch preparation according to claim 5, wherein the transdermal absorption promoter comprises N-laurylpyrrolidone and isopropyl myristate.

7. The transdermal absorption-type patch preparation according to any one of claims 1 to 6, wherein the (meth)acrylic copolymer having a pyrrolidone group is a 2-ethylhexyl acrylate-vinylpyrrolidone copolymer, a hydroxyethyl acrylate-vinylpyrrolidone copolymer, a butyl acrylate-vinylpyrrolidone copolymer, a cyclohexyl acrylate-vinylpyrrolidone copolymer, a 2-ethylhexyl methacrylate-vinylpyrrolidone copolymer, a cyclohexyl methacrylate-vinylpyrrolidone copolymer, a 2-ethylhexyl acrylate-methylvinylpyrrolidone copolymer, a 2-ethylhexyl acrylate-hydroxyethyl acrylate-vinylpyrrolidone copolymer, a 2-ethylhexyl acrylate-acrylic acid-vinylpyrrolidone copolymer, a butyl acrylate-acrylic acid-vinylpyrrolidone copolymer or a 2-ethylhexyl acrylate-acrylic acid-methylvinylpyrrolidone copolymer.

8. The transdermal absorption-type patch preparation according to claim 7, wherein the (meth)acrylic copolymer having a pyrrolidone group is a 2-ethylhexyl acrylate-vinylpyrrolidone copolymer.

9. The transdermal absorption-type patch preparation according to any one of claims 4 to 8, wherein the (meth)acrylic copolymer having a carboxyl group is an acrylic acid-octyl acrylate copolymer, a 2-ethylhexyl acrylate-vinyl acetate-acrylic acid copolymer, a 2-ethylhexyl acrylate-methyl acrylate-acrylic acid-glycidyl methacrylate copolymer, an acrylic acid-butyl acrylate copolymer, an acrylic acid-hydroxyethyl acrylate copolymer, or a 2-ethylhexyl acrylate-vinyl acetate-butyl acrylate-acrylic acid copolymer.

10. The transdermal absorption-type patch preparation according to claim 9, wherein the (meth)acrylic copolymer having a carboxyl group is an acrylic acid-octyl acrylate copolymer.

11. The transdermal absorption-type patch preparation according to any one of claims 1 to 10, wherein a content of the zonisamide or the alkali metal salt thereof in the transdermal absorption-type patch preparation is 1% by mass to 20% by mass, based on a total amount of the adhesive layer in terms of zonisamide.

12. The transdermal absorption-type patch preparation according to any one of claims 1 to 11, wherein a content of the adhesive agent in the transdermal absorption-type patch preparation is 30% by mass to 98% by mass, based on the total amount of the adhesive layer.

13. The transdermal absorption-type patch preparation according to any one of claims 1 to 12, wherein a content of the transdermal absorption promoter in the transdermal absorption-type patch preparation is 1% by mass to 40% by mass, based on the total amount of the adhesive layer.

14. The transdermal absorption-type patch preparation according to any one of claims 1 to 13, wherein a compounding amount of the adhesive agent in the transdermal absorption-type patch preparation is 1.5 parts by mass to 40 parts by mass, based on 1 part by mass of the zonisamide or the alkali metal salt thereof.

15. The transdermal absorption-type patch preparation according to any one of claims 1 to 14, wherein a compounding amount of the transdermal absorption promoter in the transdermal absorption-type patch preparation is 0.05 parts by mass to 40 parts by mass, based on 1 part by mass of the zonisamide or the alkali metal salt thereof.

16. The transdermal absorption-type patch preparation according to claim 1, wherein the adhesive agent comprises a 2-ethylhexyl acrylate-vinylpyrrolidone copolymer and an acrylic acid-octyl acrylate copolymer, and
the transdermal absorption promoter comprises N-laurylpyrrolidone and isopropyl myristate, the compounding amount of the adhesive agent is 10 parts by mass to 25 parts by mass, and the compounding amount of the transdermal absorption promoter is 1 part by mass to 10 parts by mass, based on 1 part by mass of the zonisamide or the alkali metal salt thereof.

17. The transdermal absorption-type patch preparation according to any one of claims 1 to 16, wherein a thickness of the adhesive layer is 30 µm to 200 µm.

## Patentansprüche

1. Pflasterpräparat vom transdermalen Absorptionstyp, umfassend eine Haftschicht, wobei die Haftschicht umfasst:
Zonisamid oder ein Alkalimetallsalz davon,
ein Haftmittel, umfassend ein (Meth)acrylcopolymer, das eine Pyrrolidongruppe aufweist, und
einen transdermalen Absorptionspromotor, umfassend N-Alkylpyrrolidon,
wobei das N-Alkylpyrrolidon N-Laurylpyrrolidon, N-Octylpyrrolidon, N-Heptylpyrrolidon, N-Hexylpyrrolidon, N-Nonylpyrrolidon, N-Decylpyrrolidon, N-Undecylpyrrolidon, N-Tridecylpyrrolidon, N-Tetradecylpyrrolidon, N-Pentadecylpyrrolidon, N-Hexadecylpyrrolidon, N-Heptadecylpyrrolidon oder N-Octadecylpyrrolidon ist.

2. Pflasterpräparat vom transdermalen Absorptionstyp nach Anspruch 1, wobei das N-Alkylpyrrolidon N-Laurylpyrrolidon oder N-Octylpyrrolidon ist.

3. Pflasterpräparat vom transdermalen Absorptionstyp nach Anspruch 2, wobei das N-Alkylpyrrolidon N-Laurylpyrrolidon ist.

4. Pflasterpräparat vom transdermalen Absorptionstyp nach einem der Ansprüche 1 bis 3, wobei das Haftmittel ein (Meth)acrylcopolymer, das eine Pyrrolidongruppe aufweist, und ein (Meth)acrylcopolymer, das eine Carboxylgruppe aufweist, umfasst.

5. Pflasterpräparat vom transdermalen Absorptionstyp nach einem der Ansprüche 1 bis 4, wobei der transdermale Absorptionspromotor N-Laurylpyrrolidon umfasst, und
mindestens ein Mitglied, ausgewählt aus der Gruppe, bestehend aus Polyethylenglykolmonolaurat, Lauromacrogol, Isopropylmyristat, Isopropylpalmitat, Oleyloleat und Hexyllaurat.

6. Pflasterpräparat vom transdermalen Absorptionstyp nach Anspruch 5, wobei der transdermale Absorptionspromotor N-Laurylpyrrolidon und Isopropylmyristat umfasst.

7. Pflasterpräparat vom transdermalen Absorptionstyp nach einem der Ansprüche 1 bis 6, wobei das (Meth)acrylcopolymer, das eine Pyrrolidongruppe aufweist, ein 2-Ethylhexyethylacrylat-Vinylpyrrolidon-Copolymer, ein Hydroxyethylacrylat-Vinylpyrrolidon-Copolymer, ein Butylacrylat-Vinylpyrrolidon-Copolymer, ein Cyclohexylacrylat-Vinylpyrrolidon-Copolymer, ein 2-Ethylhexylmethacrylat-Vinylpyrrolidon-Copolymer, ein Cyclohexylmethacrylat-Vinylpyrrolidon-Copolymer, ein 2-Ethylhexylacrylat-Methylvinylpyrrolidon-Copolymer, ein 2-Ethylhexylacrylat-Hydroxyethylacrylat-Vinylpyrrolidon-Copolymer, ein 2-Ethylhexylacrylat-Acrylsäure-Vinylpyrrolidon-Copolymer, ein Butylacrylat-Acrylsäure-Vinylpyrrolidon-Copolymer oder ein 2-Ethylhexylacrylat-Acrylsäure-Methylvinylpyrrolidon-Copolymer ist.

8. Pflasterpräparat vom transdermalen Absorptionstyp nach Anspruch 7, wobei das (Meth)acrylcopolymer, das eine Pyrrolidongruppe aufweist, ein 2-Ethylhexylacrylat-Vinylpyrrolidon-Copolymer ist.

9. Pflasterpräparat vom transdermalen Absorptionstyp nach einem der Ansprüche 4 bis 8, wobei das (Meth)acrylcopolymer, das eine Carboxylgruppe aufweist, ein Acrylsäure-Octylacrylat-Copolymer, ein 2-Ethylhexylacrylat-Vinylacetat-Acrylsäure-Copolymer, ein 2-Ethylhexylacrylat-Methylacrylat-Acrylsäure-Glycidylmethacrylat-Copolymer, ein Acrylsäure-Butylacrylat-Copolymer, ein Acrylsäure-Hydroxyethylacrylat-Copolymer oder ein 2-Ethylhexylacrylat-Vinylacetat-Butylacrylat-Acrylsäure-Copolymer ist.

10. Pflasterpräparat vom transdermalen Absorptionstyp nach Anspruch 9, wobei das (Meth)acrylcopolymer, das eine Carboxylgruppe aufweist, ein Acrylsäure-Octylacrylat-Copolymer ist.

11. Pflasterpräparat vom transdermalen Absorptionstyp nach einem der Ansprüche 1 bis 10, wobei ein Gehalt des Zonisamids oder des Alkalimetallsalzes davon in dem Pflasterpräparat vom transdermalen Absorptionstyp 1 Masse-% bis 20 Masse-% beträgt, bezogen auf die Gesamtmenge der Haftschicht, bezogen auf Zonisamid.

12. Pflasterpräparat vom transdermalen Absorptionstyp nach einem der Ansprüche 1 bis 11, wobei ein Gehalt des Haftmittels in dem Pflasterpräparat vom transdermalen Absorptionstyp 30 Masse-% bis 98 Masse-%, bezogen auf die Gesamtmenge der Haftschicht, beträgt.

13. Pflasterpräparat vom transdermalen Absorptionstyp nach einem der Ansprüche 1 bis 12, wobei ein Gehalt des transdermalen Absorptionspromotors in dem Pflasterpräparat vom transdermalen Absorptionstyp 1 Masse-% bis 40 Masse-%, bezogen auf die Gesamtmenge der Haftschicht, beträgt.

14. Pflasterpräparat vom transdermalen Absorptionstyp nach einem der Ansprüche 1 bis 13, wobei eine Compoundierungsmenge des Haftmittels in dem Pflasterpräparat vom transdermalen Absorptionstyp 1,5 Massenteile bis 40 Massenteile beträgt, bezogen auf 1 Massenteil des Zonisamids oder des Alkalimetallsalzes davon.

15. Pflasterpräparat vom transdermalen Absorptionstyp nach einem der Ansprüche 1 bis 14, wobei eine Compoundierungsmenge des transdermalen Absorptionspromotors in dem Pflasterpräparat vom transdermalen Absorptionstyp 0,05 Massenteile bis 40 Massenteile beträgt, bezogen auf 1 Massenteil des Zonisamids oder des Alkalimetallsalzes davon.

16. Pflasterpräparat vom transdermalen Absorptionstyp nach Anspruch 1, wobei das Haftmittel ein 2-Ethylhexylacrylat-Vinylpyrrolidon-Copolymer und ein Acrylsäure-Octylacrylat-Copolymer umfasst, und
der transdermale Absorptionspromotor N-Laurylpyrrolidon und Isopropylmyristat umfasst, die Compoundierungsmenge des Haftmittels 10 Massenteile bis 25 Massenteile beträgt und die Compoundierungsmenge des transdermalen Absorptionspromotors 1 Massenteil auf 10 Massenteile beträgt, bezogen auf 1 Massenteil des Zonisamids oder des Alkalimetallsalzes davon.

17. Pflasterpräparat vom transdermalen Absorptionstyp nach einem der Ansprüche 1 bis 16, wobei die Dicke der Haftschicht 30 mm bis 200 mm beträgt.

## Revendications

1. Préparation de timbre de type absorption transdermique comprenant une couche adhésive, la couche adhésive comprenant
du zonisamide ou un sel de métal alcalin de celui-ci,
un agent adhésif comprenant un copolymère (méth)acrylique ayant un groupe pyrrolidone, et
un promoteur d'absorption transdermique comprenant de la N-alkylpyrrolidone, dans laquelle la N-alkylpyrrolidone est la N-laurylpyrrolidone, N-octylpyrrolidone, N-heptylpyrrolidone, N-hexylpyrrolidone, N-nonylpyrrolidone, N-décylpyrrolidone, N-undécylpyrrolidone, N-tridécylpyrrolidone, N-tétradécylpyrrolidone, N-pentadécylpyrrolidone, N-hexadécylpyrrolidone, N-heptadécylpyrrolidone ou N-octadécylpyrrolidone.

2. Préparation de timbre de type absorption transdermique selon la revendication 1, dans laquelle la N-alkylpyrrolidone est la N-laurylpyrrolidone ou N-octylpyrrolidone.

3. Préparation de timbre de type absorption transdermique selon la revendication 2, dans laquelle la N-alkylpyrrolidone est la N-laurylpyrrolidone.

4. Préparation de timbre de type absorption transdermique selon l'une quelconque des revendications 1 à 3, dans laquelle l'agent adhésif comprend un copolymère (méth)acrylique ayant un groupe pyrrolidone et un copolymère (méth)acrylique ayant un groupe carboxyle.

5. Préparation de timbre de type absorption transdermique selon l'une quelconque des revendications 1 à 4, dans laquelle le promoteur d'absorption transdermique de la N-laurylpyrrolidone, et
au moins un membre sélectionné parmi le groupe constitué du monolaurate de polyéthylèneglycol, lauromacrogol, myristate d'isopropyle, palmitate d'isopropyle, oléate d'oléyle et laurate d'hexyle.

6. Préparation de timbre de type absorption transdermique selon la revendication 5, dans laquelle le promoteur d'absorption transdermique comprend de la N-laurylpyrrolidone et du myristate d'isopropyle.

7. Préparation de timbre de type absorption transdermique selon l'une quelconque des revendications 1 à 6, dans laquelle le copolymère (méth)acrylique ayant un groupe pyrrolidone est un copolymère d'acrylate de 2-éthylhexyle-vinylpyrrolidone, un copolymère d'acrylate d'hydroxyéthyle-vinylpyrrolidone, un copolymère d'acrylate de butyle-vinylpyrrolidone, un copolymère d'acrylate de cyclohexyle-vinylpyrrolidone, un copolymère de méthacrylate de 2-éthylhexyle-vinylpyrrolidone, un copolymère de méthacrylate de cyclohexyle-vinylpyrrolidone, un copolymère d'acrylate de 2-éthylhexyleméthylvinylpyrrolidone, un copolymère d'acrylate de 2-éthylhexyle-acrylate d'hydroxyéthyle-vinylpyrrolidone, un copolymère d'acrylate de 2-éthylhexyle-acide acrylique-vinylpyrrolidone, un copolymère d'acrylate de butyle-acide acrylique-vinylpyrrolidone ou un copolymère d'acrylate de 2-éthylhexyle-acide acrylique-méthylvinylpyrrolidone.

8. Préparation de timbre de type absorption transdermique selon la revendication 7, dans laquelle le copolymère (méth)acrylique ayant un groupe pyrrolidone est un copolymère d'acrylate de 2-éthylhexyle-vinylpyrrolidone.

9. Préparation de timbre de type absorption transdermique selon l'une quelconque des revendications 4 à 8, dans laquelle le copolymère (méth)acrylique ayant un groupe carboxyle est un copolymère d'acide acrylique-acrylate d'octyle, un copolymère d'acrylate de 2-éthylhexyle-acétate de vinyle-acide acrylique, un copolymère d'acrylate de 2-éthylhexyle-acrylate de méthyle-acide acrylique-méthacrylate de glycidyle, un copolymère d'acide acrylique-acrylate de butyle, un copolymère d'acide acrylique-acrylate d'hydroxyéthyle ou un copolymère d'acrylate de 2-éthylhexyle-acétate de vinyle-acrylate de butyle-acide acrylique.

10. Préparation de timbre de type absorption transdermique selon la revendication 9, dans laquelle le copolymère (méth)acrylique ayant un groupe carboxyle est un copolymère d'acide acrylique-acrylate d'octyle.

11. Préparation de timbre de type absorption transdermique selon l'une quelconque des revendications 1 à 10, dans laquelle une teneur en le zonisamide ou le sel de métal alcalin de celui-ci dans la préparation de timbre de type absorption transdermique est de 1 % en masse à 20 % en masse, basée sur une quantité totale de la couche adhésive en termes de zonisamide.

12. Préparation de timbre de type absorption transdermique selon l'une quelconque des revendications 1 à 11, dans laquelle une teneur en l'agent adhésif dans la préparation de timbre de type absorption transdermique est de 30 % en masse à 98 % en masse, basée sur la quantité totale de la couche adhésive.

13. Préparation de timbre de type absorption transdermique selon l'une quelconque des revendications 1 à 12, dans laquelle une teneur en le promoteur d'absorption transdermique dans la préparation de timbre de type absorption transdermique est de 1 % en masse à 40 % en masse, basée sur la quantité totale de la couche adhésive.

14. Préparation de timbre de type absorption transdermique selon l'une quelconque des revendications 1 à 13, dans laquelle une quantité de mélange de l'agent adhésif dans la préparation de timbre de type absorption transdermique est de 1,5 partie en masse à 40 parties en masse, basée sur 1 partie en masse du zonisamide ou du sel de métal alcalin de celui-ci.

15. Préparation de timbre de type absorption transdermique selon l'une quelconque des revendications 1 à 14, dans laquelle une quantité de mélange du promoteur d'absorption transdermique dans la préparation de timbre de type absorption transdermique est de 0,05 partie en masse à 40 parties en masse, basée sur 1 partie en masse du zonisamide ou du sel de métal alcalin de celui-ci.

16. Préparation de timbre de type absorption transdermique selon la revendication 1, dans laquelle l'agent adhésif comprend un copolymère d'acrylate de 2-éthylhexyle-vinylpyrrolidone et un copolymère d'acide acrylique-acrylate d'octyle, et le promoteur d'absorption transdermique comprend de la N-laurylpyrrolidone et du myristate d'isopropyle, la quantité de mélange de l'agent adhésif est de 10 parties en masse à 25 parties en masse, et la quantité de mélange du promoteur d'absorption transdermique est de 1 partie en masse à 10 parties en masse, basée sur 1 partie en masse du zonisamide ou du sel de métal alcalin de celui-ci.

17. Préparation de timbre de type absorption transdermique selon l'une quelconque des revendications 1 à 16, dans laquelle une épaisseur de la couche adhésive est de 30 µm à 200 µm.
